# EUROPEAN PATENT APPLICATION

(11) **EP 0 790 241 A1**
(43) Date of publication of application: **20.08.1997**
(21) Application number: 96916180.1
(22) Date of filing: 11.06.1996
(51) Int. Cl.: C07D 231/12, A61K 49/00, C07D 231/56

(54) **COMPLEXONES WITH THE STRUCTURE OF N-2-(AZOL-1(2)-YL)ETHYLIMINODIACETIC ACIDS, SYNTHESIS, ANALYTICAL STUDY AND BIOLOGICAL APPLICATIONS**

(30) Priority: 13.06.1995 ES 19950001185
(71) Applicant: UNIVERSIDAD NACIONAL DE EDUCACION A DISTANCIA, 28015 Madrid (ES); CONSEJO SUPERIOR DE INVESTIGACIONES CIENTIFICAS, E-28006 Madrid (ES)
(72) Inventor: BALLESTEROS GARCIA, Paloma, U.N.E.D., E-28040 Madrid (ES); LOPEZ LARRUBIA, Pilar, U.N.E.D., E-28040 Madrid (ES); CERDAN GARCIA-ESTELLER, Sebastian, C.S.I.C., E-28006 Madrid (ES)
(74) Representative: Elzaburu Marquez, Alberto, et al
(86) International application number: PCT/ES1996/000129
(87) International publication number: WO 1996/041797

(57) **Abstract**

Complexones having the structure of N-2-(azol-1(2)-yl(ethyl-iminodiacetic acids, synthesis, analytical study and biological applications, **characterized by** their complexing capacity over Ca²⁺, Mg²⁺ ions and other bivalent cations, lanthanides and transition metals. In the formula azolN-CH₂-CH₂-N(CH₂-CO₂R)₂ (I), azol is pirazol; 3,5-dimethylpirazol; indazol; etc. and R is alkyl or H or Na. The compounds having the general formula (I), are obtained by an alkylation reaction of methyl iminodiacetate with N-bromoethylazols and further hydrolysis in acid or basic medium. The process may be modified by alternative synthetic processes which involve N-aminoethylazols as starting materials or the use of cyclization processes in the preparation of N-substituted azol. They can be applied in spectroscopy and nuclear magnetic resonance (NMR) imaging of hydrogen or other nuclei as extrinsic probes of Ca²⁺, Mg²⁺ or other metal ions and as contrast agents, in solutions, biological extracts, tissues, entire animals and human beings.

## Description

**Introduction:** The biomedical applications of nuclear magnetic resonance (NMR) have undergone very considerable development in the last decade¹. Both spectroscopy (MRS) and imaging (MRI) have been employed in order to study non-invasively a multitude of physiological and pathological processes in animals and human beings². More recently, it has been possible to extend the applications of NMR to the study of the physiology and pathology of eukaryotic cells and cell cultures³. These advances have enabled a start to be made in studying fundamental aspects of biology and medicine, such as proliferation, differentiation and cell development, by NMR. In all these processes, significant changes take place in the intracellular concentration of calcium (pCaᵢ)⁴. In fact, the pCaᵢ or alterations thereof play(s) a fundamental part in a wide variety of biological processes, such as muscle contraction⁵, transduction of the hormonal message⁶, platelet aggregation and coagulation⁷, exocytosis of neurotransmitters⁸, phosphorylation of proteins⁹, glycogen metabolism¹⁰ and phototransduction and adaptation of photoreceptors¹¹.

The concentration of Ca²⁺ in the extracellular medium is 10⁵ times as high as its intracellular concentration, and small changes in the permeability of the membrane cause drastic changes in the intracellular Ca²⁺ concentration. The result of this is that many hormonal signals utilize variations in the intracellular Ca²⁺ concentration as mechanisms of transduction of the message across the membrane^{6,12}.

In view of the fact that intracellular Ca²⁺ concentrations are not directly observable by NMR, it has been necessary to use a series of detectors (or probes) which, due to their chelating capacity, enable the pCaᵢ to be determined indirectly using magnetic resonance. Hitherto, the majority of pCaᵢ measurements carried out by NMR have almost always utilized ¹⁹F NMR, employing fluorinated analogues of BAPTA (bis(o-aminophenoxy)ethane-N,N,N',N'-tetraacetic acid) as extrinsic probes^{13,14}.

¹H NMR, in spite of being the most sensitive and conventional of NMR spectroscopies, still remains hardly used for any of these purposes. Accordingly, we have proposed developing a new series of extrinsic probes for pCaᵢ determination by ¹H NMR.

**Prior art:** The Ca²⁺-chelating agents most used in biological systems consist of molecules known as complexones which contain iminodiacetic acid (N-carboxymethylglycine) units¹⁵. There are other types of molecules capable of complexing with calcium, such as, for example, crown ethers, but, owing to their ionophore or toxic character, they cannot be used as detectors in cellular systems. Among the wide variety of complexones described in the literature, only ethylenediaminetetraacetic acid (EDTA) has been used previously as an extrinsic probe for pCa by ¹H NMR in chromaffin granules¹⁶. The method is based on the fact that the rates of exchange between free Ca²⁺ and the EDTA-Ca²⁺ complex are less than 360 s⁻¹, which is consequently in the region of slow exchange on the ¹H NMR time scale. The outcome is that, in high resolution spectrometers, resonances from free EDTA (FR) and from its chelated forms with metals (BD) can be observed simultaneously (Figure 1). It should be emphasized that the chelated forms with Ca²⁺ and Mg²⁺ give resonances with a different chemical shift, so that it is possible to distinguish between EDTA-CA²⁺ and EDTA-Mg²⁺ complexes.

(HOOCCH₂)₂NCH₂-CH₂N(CH₂CO₂H)₂ EDTA

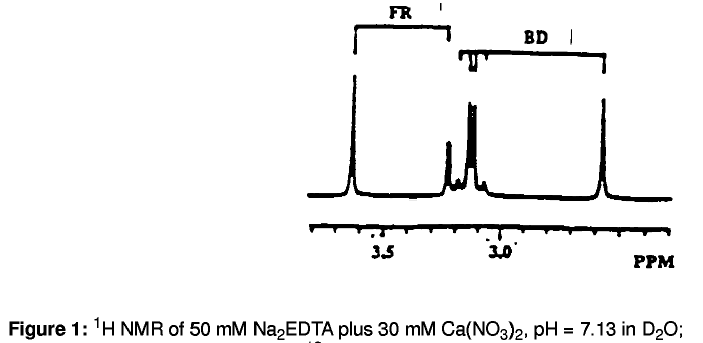

Nevertheless, EDTA has the drawback that the resonances in which the effect of chelation is detected appear in the region from 2.6-3.6 ppm, one of the most complex regions of the ¹H NMR spectrum of biological systems.

Moreover, the chelation of Ca²⁺ with BAPTA and EGTA (ethylene glycol bis(β-aminoethyl ether)-N,N,N',N'-tetraacetic acid), agents commonly used in potentiometry or fluorescence, takes place in the region of rapid exchange (on the NMR scale) and, consequently, it is not possible to distinguish the free and bound forms or the type of cation linked to the complexone. Accordingly, it has not been possible to use these compounds directly as extrinsic probes for pCa by ¹H NMR¹⁷.

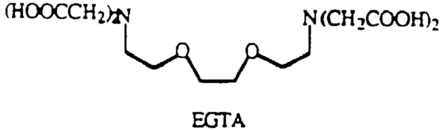

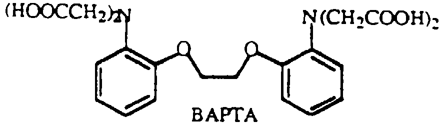

For this reason, fluorinated derivatives of BAPTA have previously been developed for use with ¹⁹F NMR¹⁸ or also as fluorescent probes¹¹³.

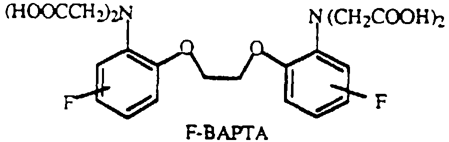

**Description:** In view of the foregoing, in the design of extrinsic detectors for pCa using ¹H NMR, the following aspects should be borne in mind: i) ¹H NMR spectra of cell extracts display their greatest complexity in the region of 1-5 ppm, in which region the majority of cellular metabolites resonate¹⁹. ii) It is consequently essential for the extrinsic detectors to contain in their structure groups which are sensitive to the variations produced by chelation of the metal, and for them, in addition, to resonate in fields below 5 ppm. For this purpose, heterocyclic ring systems prove very suitable, and especially azoles. These heterocyclic nuclei give rise to well resolved and readily identifiable signals in the region from 6 to 8 ppm. iii) Now, in order to be able to be used as an extrinsic probe which registers exclusively changes due to the chelation of metals but not to pH variations, the pKₐ of the heterocyclic ring system should be remote from physiological pH. Ruling out imidazole (pKₐ = 6.9-7.0), pyrazole (pKₐ = 2.5), 3,5-dimethylpyrazole (pKₐ = 4.2), 1,2,4-triazole (pKₐ = 2.2) and indazole (pKₐ = 1.3) are suitable candidates.

Consequently, the ideal complexones for determining pCa by ¹H NMR should combine iminodiacetic acid units capable of chelating Ca²⁺ (pCa) and heterocyclic ring systems according to the general structure:

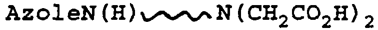

From the standpoint of design of the probe, the anchoring of the iminodiacetic acid to the azole may be carried out in various ways. In this work, we have opted to do this via an alkyl chain of two carbon atoms linked to the N¹ nitrogen atom. The following diagram illustrates the case of the complexone derived from pyrazole.

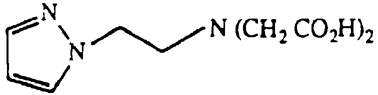

The present invention covers the following objectives: 1: To synthesize complexones with an iminodiacetic acid unit linked via an alkyl chain of two carbon atoms to the N¹ (or N²) nitrogen atom of an azole ring system; 2: To study by ¹H NMR the behaviour of the azole protons of these molecules in relation to pH variations; and 3: To study by ¹H NMR the behaviour of the azole protons of the said molecules in relation to the addition of excess Ca²⁺ in order to establish their viability as potential probes for pcaᵢ, and in relation to the addition of excess Mg²⁺ in order to determine the extent to which this cation present in cells may interfere in the measurements of calcium.

The products synthesized during this work are presented below:

### 1(2)-(2-Bromoethyl)azoles

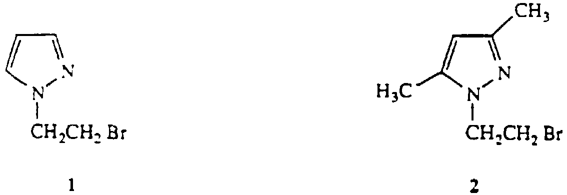

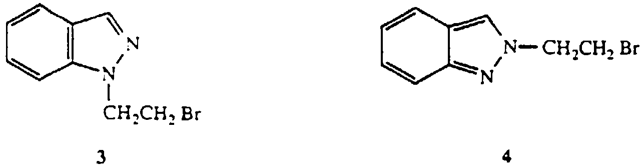

### Methyl N-[2-(1 (2)-azolyl)ethyl]iminodiacetates or methyl N-(methoxycarbonylmethyl)-N-[2-(1(2)- azolyl)ethyl]glycinates

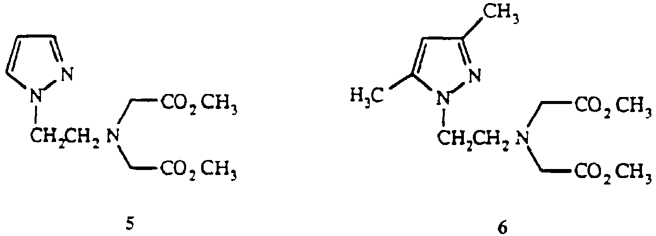

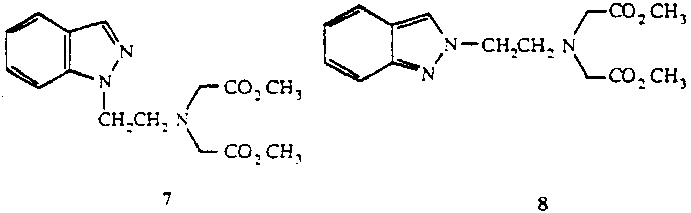

### N-[2-(1-Pyrazolyl)ethyl]iminodiacetic acid hydrochloride or N-(carboxymethyl)-N-[2-(1-pyrazolyl)ethyl]glycine hydrochloride

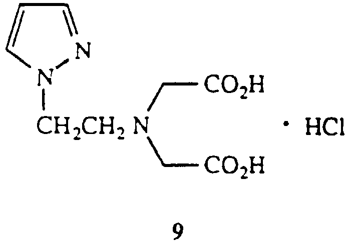

### Sodium N-[2-(1(2)-azolyl)ethyl]iminodiacetates or methyl N-(carboxymethyl)-N-[2-(1(2)-azolyl)ethyl]glycinate disodium salts

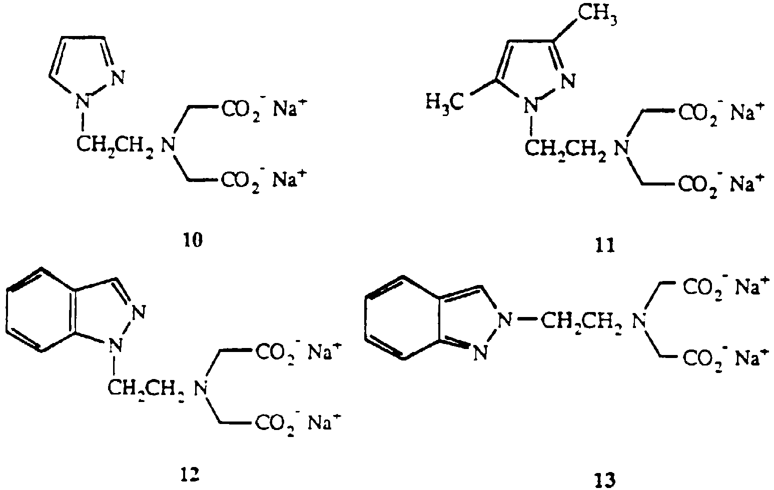

The synthesis of the abovementioned complexones was carried out according to the following retrosynthetic scheme:

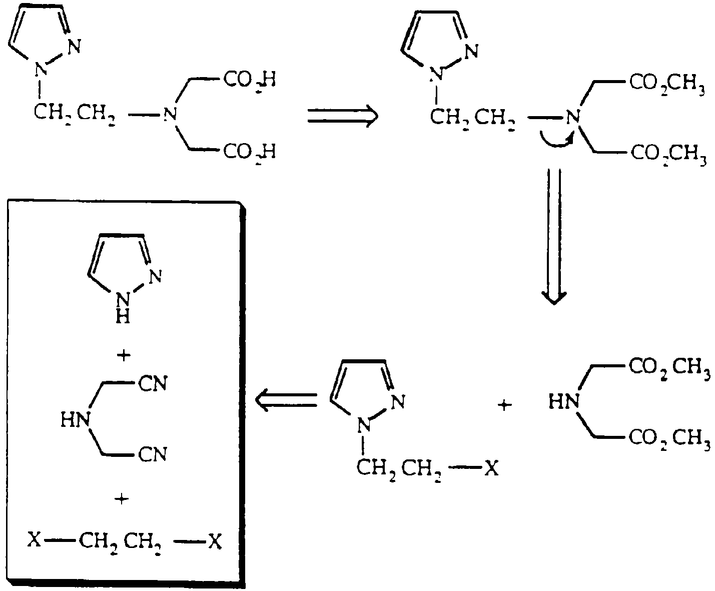

Production of the bromoethyl derivatives 1-4 was carried out starting from the corresponding azole and 1,2-dibro-moethane using the technique of liquid-liquid phase transfer catalysis (PTC)²²,²³, employing 40% NaOH, tetrabutylammonium bromide (TBAB) as catalyst and an excess of 1,2-dibromoethane as solvent.

1-(2-Bromoethyl)pyrazole 1 had been synthesized previously employing tetrabutylammonium sulphate as catalyst and maintaining the reaction for 1 hour under vigorous reflux²⁴. In this work, these and other conditions were tried with different starting azoles in order to arrive in this way at the most optimal method of preparing the 2-bromoethyl derivative from each of them. From the reaction of the azole with 1,2-dibromoethane, three different products may, in principle, be obtained: the desired 2-bromoethylazole; the N,N'-bis(azolyl)ethane resulting from double alkylation with 1,2-dibro-moethane; and the vinylazole as elimination product.

The proportion in which each of these is formed depends on the stoichiometry of the reaction. The use of an excess of 1,2-dibromoethane will potentially lead to an increase in 2-bromoethylazole which will be obtained as the preponderant product. Moreover, control of the reaction temperature and time will contribute to a decrease in the formation both of the elimination product and of the bis derivative.

Thus, by following this synthetic scheme and employing the reagent 1,2-dibromoethane as solvent in an 8- to 10-fold excess with respect to the corresponding heterocycle, success was achieved in optimizing the production of the different bromoethylazoles.

The corresponding vinyl and bis derivatives were identified in the ¹H NMR spectra of the crude reaction products by their characteristic resonances, which coincide with the data described in the literature for the said compounds²⁵.

These products were purified by column chromatography, followed in some cases by vacuum distillation. Although the vinyl azole cannot be separated completely from the bromoethyl derivative formed in every case, this does not represent a problem for the synthesis of the final complexone, since it does not interfere in any way in the next step, following which it proves readily separable from the rest of the products.

The alkylation of the methyl iminodiacetate²⁷ with the 2-bromoethylazoles **1-4** required a basic species which would neutralize the HBr liberated. It was not possible to use the PTC technique because the basic medium produced a hydrolysis of the ester groups. For this reason, it was necessary to employ an amine with which the hydrobromide derived from the latter would form. Methyl iminodiacetate itself was used, our decision being based on a previous report in the literature in which chloroacetonitrile was anchored to the iminodiacetate²⁸.

In this way, the synthesis of the different methyl azolylethyliminodiacetates was carried out by direct reaction of two equivalents of iminodiacetate, compound **14,** per equivalent of 2-bromoethylazole, one of which produces the nucleophilic substitution and the other neutralizes the HBr forming the hydrobromide.

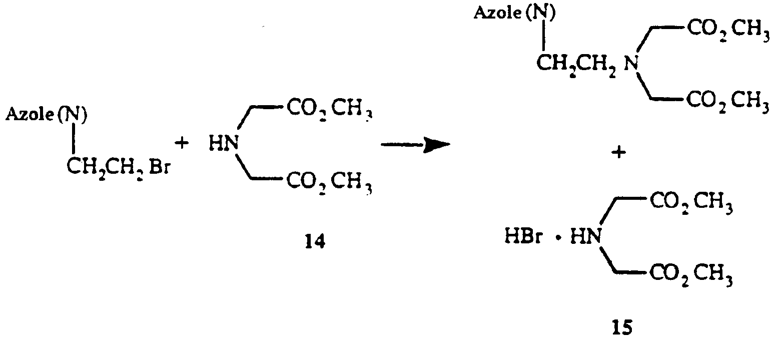

When the reaction is complete, the ammonium salt **15** may be recovered and the base regenerated with K₂CO₃.

The synthesis of the derived azolylethyliminodiacetates was verified for all the bromoethylazoles by maintaining the reaction at 110°C for a period of time of from 2.5 h to 4.5 h, according to the starting 1 (2)-(2-bromoethyl)azole, similar yields, between 55% and 65%, being obtained in all cases.

Complete purification of these products was achieved by means of column chromatography of the crude reaction product followed by vacuum distillation. In this way, they are separated completely from the methyl iminodiacetate, whose presence interferes greatly in the next step.

The free complexones were obtained by hydrolysis of the ester groups in order to obtain the free carboxylates which are the true ion-chelating agents. This reaction may be carried out in an acidic medium (heating in excess 2N HCl) or basic medium (with an equimolecular amount of 0.6% NaOH and at r.t.). In either case, completion of the reaction is readily verified by ¹H NMR, by the non-appearance of the most intense singlet in the spectrum corresponding to the 6 isochronous protons of the two -O-CH₃ groups.

With methyl N-[2-(1-pyrazolyl)ethyl]iminodiacetate **5,** both possibilities were carried out. As acid hydrolysis product, the hydrochloride derivative **9,** strongly hygroscopic in nature and very difficult to recrystallize, was obtained. Basic hydrolysis yielded the highly stable disodium salt **10,** sufficiently pure not to require subsequent recrystallization.

With the remaining derived esters, only basic hydrolysis was carried out as the route for obtaining the complexones. By maintaining reaction for 24 h at r.t., the sodium azolylethyliminodiacetates, compounds **10-13,** were obtained in an ∼ 100% yield.

The final objective of the synthesis of the complexones **9-13** is to use them as ¹H NMR detectors for the determination of pCa and pMg, or contrast agents for MRI in cellular systems. To arrive at this point, it is essential to carry out beforehand a series of analytical tests, which are the ones presented below.

The determinations were carried out employing ¹H NMR, beginning with a pH titration in order to establish the variations in its/their chemical shift undergone by the azole proton(s) which it is desired to use in order to detect the modifications in the concentration of metal ions. It is necessary for the said protons to maintain a practically constant δ over the pH range so that any variation occurring in the latter in the presence of cations is due exclusively to the concentration of these cations in the medium.

Starting from 25 mM solutions of compounds **9** and **11-13** in D₂O and modifying the pH of different aliquots, the chemical shift of each of the protons, both azole and aliphatic, was determined for the different pH values in the range from 1 to 12. The spectra were obtained at 360.13 MHz.

The complexones used for these determinations were in all cases the sodium N-[2-(1 (2)-azolyl)ethyl]iminodiacetates, except for the one derived from the pyrazole nucleus, in which N-[2-(1-pyrazolyl)ethyl]iminodiacetic acid hydrochloride, compound 9, was used. The δ values for each proton are shown in Tables 1-4.

**TABLE 1**

| Chemical shifts (ppm) of the protons of compound **9** at different pH values. | | | | | | |
|---|---|---|---|---|---|---|
| **pH** | **H5** | **H3** | **H4** | **azole CH₂-N-** | **CH₂-N- (CH₂)** | **N-CH₂-CO** |
| **1.26** | 7.79 | 7.71 | 6.45 | 4.69 | 3.87 | 4.09 |
| **2.35** | 7.76 | 7.68 | 6.45 | 4.66 | 3.77 | 3.85 |
| **3.84** | 7.75 | 7.68 | 6.44 | 4.66 | 3.74 | 3.77 |
| **7.41** | 7.75 | 7.66 | 6.43 | 4.60 | 3.65 | 3.68 |
| **8.28** | 7.73 | 7.62 | 6.39 | 4.46 | 3.38^{a} | 3.45 |
| **11.26** | 7.73 | 7.58 | 6.35 | 4.30 | 3.06 | 3.17 |

| | | | | | | |
|---|---|---|---|---|---|---|
| ^{a} Broadened signal. | | | | | | |

**TABLE 2**

| Chemical shifts (ppm) of the protons of compound **11** at different pH values. | | | | | | |
|---|---|---|---|---|---|---|
| **pH** | **H4** | **azole CH₂-N-** | **CH₂-N-(CH₂)** | **N-CH₂-CO** | **CH₃(3)** | **CH₃(5)** |
| **2.35** | 6.07 | 4.46 | 3.61 | 3.81 | 2.27 | 2.16 |
| **3.10** | 6.01 | 4.44 | 3.60 | 3.74 | 2.25 | 1.71 |
| **3.99** | 5.99 | 4.43 | 3.59 | 3.72 | 2.24 | 2.15 |
| **5.32** | 5.99 | 4.43 | 3.58 | 3.72 | 2.25 | 2.15 |
| **6.18** | 6.02 | 4.46 | 3.61 | 3.74 | 2.27 | 2.18 |
| **6.90** | 5.98 | 4.41^{a} | 3.56^{a} | 3.69 | 2.24 | 2.14 |
| **7.98** | 5.99 | 4.35^{a} | 3.51^{a} | 3.64 | 2.26 | 2.17 |
| **9.15** | 5.90 | 4.17^{a} | 3.04^{a} | 3.31^{a} | 2.25 | 2.15 |
| **10.11** | 5.89 | 4.08 | 2.90 | 3.20 | 2.21 | 2.11 |
| **11.79** | 5.92 | 4.10 | 2.91 | 3.22 | 2.24 | 2.14 |
| **13.32** | 5.89 | 4.07 | 2.99 | 3.19 | 2.21 | 2.11 |

| | | | | | | |
|---|---|---|---|---|---|---|
| ^{a} Broadened signal or signals. | | | | | | |

**TABLE 3**

| Chemical shift (ppm) of the aromatic protons of compound **12** at different pH values. | | | | | |
|---|---|---|---|---|---|
| **pH** | **H3** | **H4** | **H7** | **H6** | **H5** |
| **2.00** | 8.22 | 7.91 | 7.63 | 7.57 | 7.30 |
| **2.73** | 8.22 | 7.90 | 7.63 | 7.56 | 7.30 |
| **3.91** | 8.22 | 7.89 | 7.62 | 7.55 | 7.30 |
| **4.75** | 8.22 | 7.89 | 7.63 | 7.56 | 7.30 |
| **6.26** | 8.22 | 7.90 | 7.63 | 7.56 | 7.30 |
| **6.94** | 8.21 | 7.89 | 7.63 | 7.55 | 7.30 |
| **8.40** | 8.17 | 7.87 | 7.65 | 7.54 | 7.27 |
| **8.91** | 8.14 | 7.86 | 7.68 | 7.53 | 7.26 |
| **9.84** | 8.12 | 7.85 | 7.66 | 7.52 | 7.25 |
| **10.92** | 8.11 | 7.85 | 7.67 | 7.52 | 7.25 |
| **12.09** | 8.11 | 7.85 | 7.67 | 7.52 | 7.25 |

**TABLE 4**

| Chemical shift (ppm) of the aromatic protons of compound **13** at different pH values. | | | | | |
|---|---|---|---|---|---|
| **pH** | **H3** | **H4** | **H7** | **H6** | **H5** |
| **1.97** | 8.37 | 7.83 | 7.71 | 7.45 | 7.22 |
| **2.85** | 8.37 | 7.83 | 7.72 | 7.45 | 7.22 |
| **4.05** | 8.37 | 7.83 | 7.72 | 7.45 | 7.22 |
| **5.43** | 8.37 | 7.83 | 7.72 | 7.45 | 7.22 |
| **6.56** | 8.37 | 7.83 | 7.72 | 7.45 | 7.22 |
| **7.12** | 8.37 | 7.83 | 7.72 | 7.45 | 7.21 |
| **7.98** | 8.37 | 7.82 | 7.70 | 7.44 | 7.20 |
| **8.72** | 8.37 | 7.82 | 7.68 | 7.42 | 7.19 |
| **9.95** | 8.37 | 7.82 | 7.67 | 7.42 | 7.19 |
| **11.70** | 8.37 | 7.82 | 7.67 | 7.42 | 7.18 |
| **12.40** | 8.37 | 7.82 | 7.67 | 7.42 | 7.17 |

It was observed that the chemical shifts of all the protons move slightly downfield as the pH value falls, with the exception of the azole proton H7 of sodium N-[2-(1-indazolyl)ethyl]iminodiacetate 12, which does the opposite. Likewise, it was established in all the complexones that the protons of the methylenes linked directly to the nitrogen which titrates vary appreciably in their δ around the value of their pKₐ (pH between 7 and 8). However, the azole protons hardly undergo any modification over the entire pH range.

The variation which occurred in the δ of the different azole protons of complexones between the two extremes of pH (1 to 12) is reflexed in Table 5.

**TABLE 5**

| Δδ (ppm) in the azole protons of compounds **9** and **11-13** over the pH range studied. | | | |
|---|---|---|---|
| **Compound 9.** | H3: + 0.13 | H4: + 0.10 | H5: + 0.06 |
| **Compound 11.** | | H4: + 0.17 | |
| **Compound 12.** | H3: + 0.11 | H4: + 0.06 | H5: + 0.05 |
| | H6: + 0.05 | H7: - 0.04 | |
| **Compound 13.** | H3: 0.00 | H4: + 0.01 | H5: + 0.05 |
| | H6: + 0.03 | H7: + 0.04 | |

The proton whose chemical shift undergoes the most modification with the variation in pH is H4 of sodium N-[2-(3,5-dimethyl-1-pyrazolyl)ethyl]iminodiacetate, compound **11,** and the one least affected is the proton H3 of sodium N-[2-(2-indazolyl)ethyl]iminodiacetate, compound **13,** whose resonance frequency does not vary at all with the pH of the medium.

Likewise, the variations produced in the chemical shift of the azole protons of compounds **10-13** (25 mM) as a consequence of adding an excess of Ca²⁺ and Mg²⁺ ions (in a 2:1 ion/complexone mole ratio) were studied by ¹H NM R.

This test was carried out at two different final pH values, following addition of the ion, in each case: one close to physiological pH and the other slightly alkaline. It should be borne in mind that the formation of the chelate between the complexone and the cation is accompanied by a fall in pH with respect to that of the solution without the said metal ion¹⁵. The δ values obtained for the azole protons for each of molecules **10-13,** at the two pH values and in the presence of Ca²⁺ and Mg²⁺ cations, are collated in Tables 6 and 7.

Table 8 shows the increases produced in the chemical shift of the protons of the compounds under study on adding the cations to the medium which is at a pH value close to physiological. The data obtained for the basic pH value appear in Table 9.

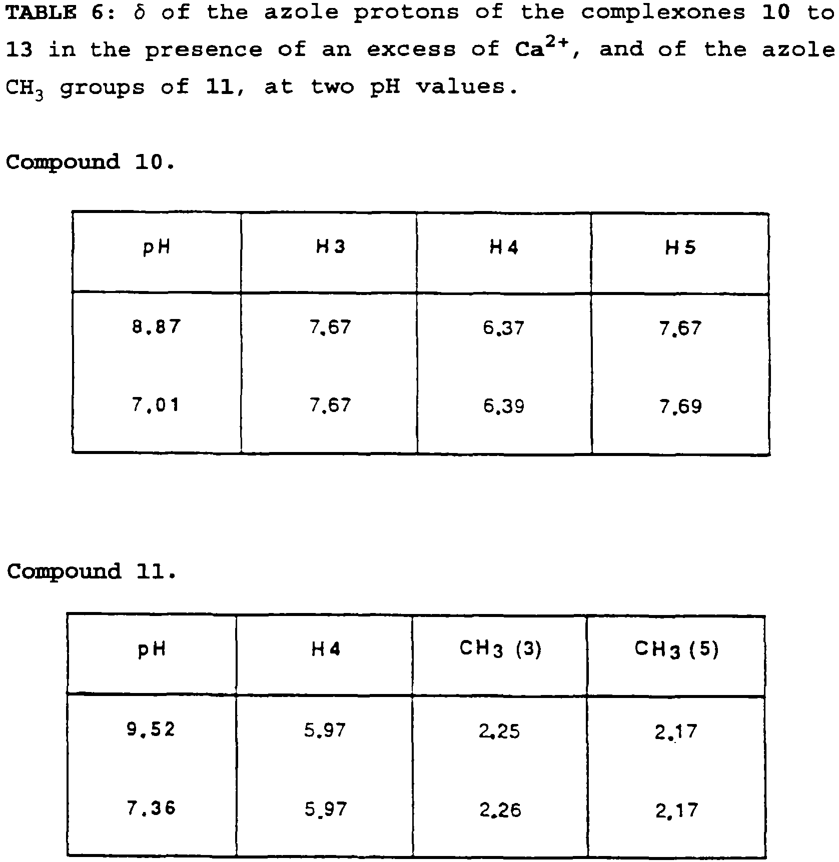

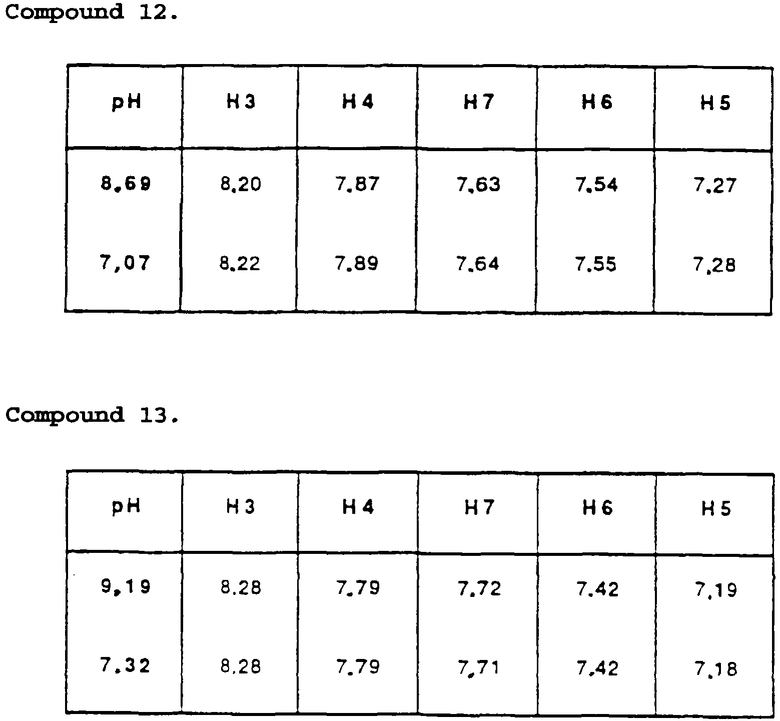

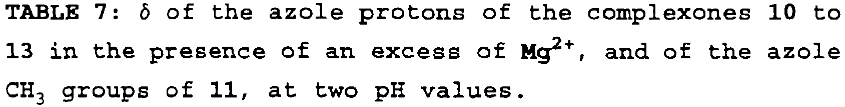

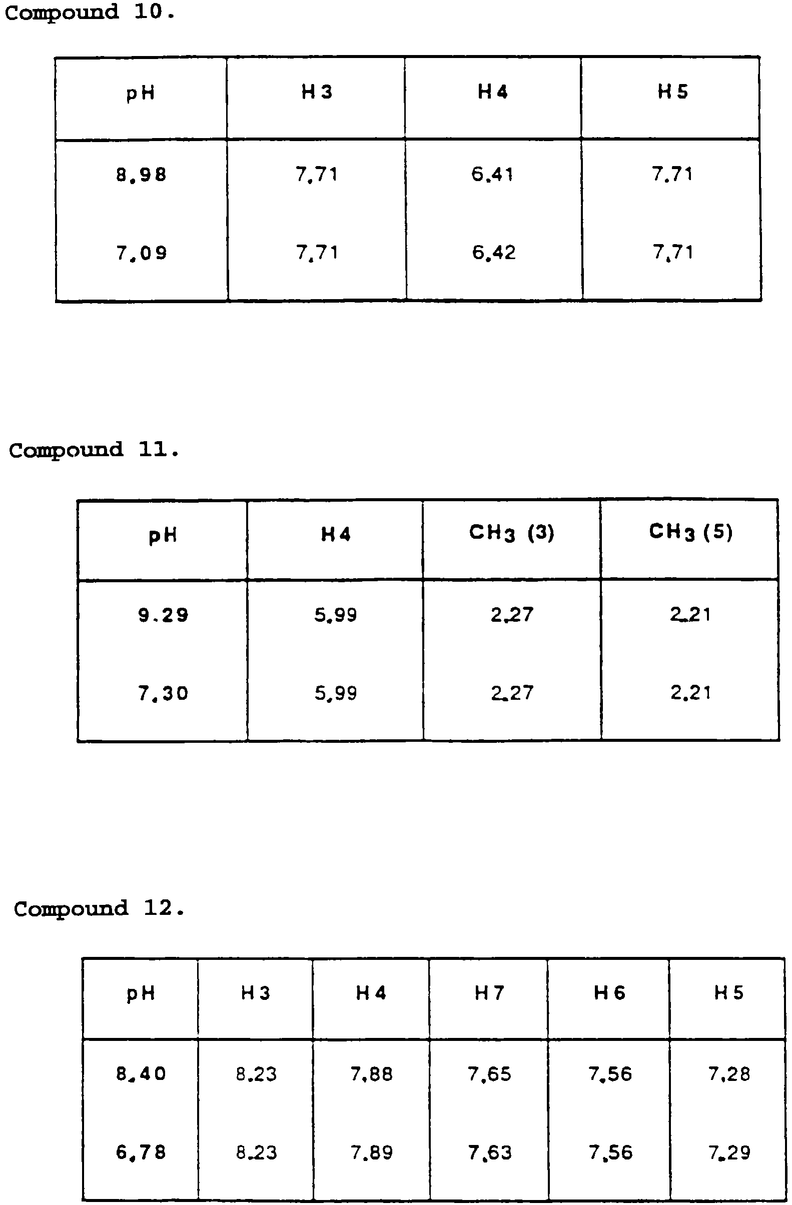

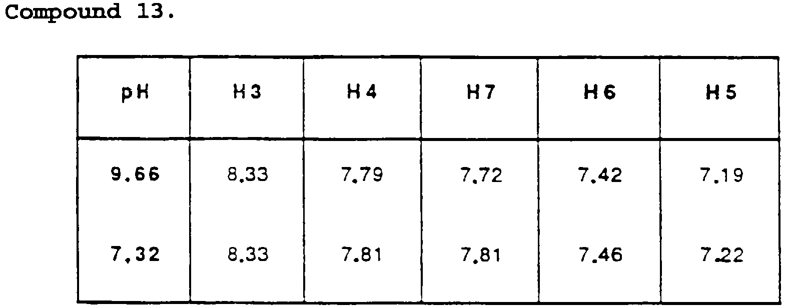

**TABLE 8**

| Δδ (ppm) which is produced in the protons under study on adding an excess of Ca²⁺ and Mg²⁺ at a pH close to physiological (∼7). | | | |
|---|---|---|---|
| **Compound** | **Protons** | **With Ca²⁺** | **With Mg²⁺** |
| 10 | H3 | +0.02 | +0.06 |
| | H4 | -0.04 | -0.02 |
| | H5 | -0.08 | -0.06 |
| 11 | H4 | +0.02 | +0.06 |
| | CH₃(3) | +0.02 | +0.04 |
| | CH₃(5) | +0.02 | +0.08 |
| 12 | H3 | +0.04 | +0.04 |
| | H4 | +0.02 | +0.02 |
| | H5 | -0.02 | -0.04 |
| | H6 | +0.02 | +0.02 |
| | H7 | +0.02 | +0.02 |
| 13 | H3 | -0.10 | -0.04 |
| | H4 | -0.07 | -0.02 |
| | H5 | -0.04 | +0.02 |
| | H6 | -0.02 | +0.02 |
| | H7 | -0.02 | +0.10 |

**TABLE 9**

| Δδ (ppm) which is produced in the protons under study on adding an excess of Ca²⁺ and Mg²⁺ at basic pH (∼9). | | | |
|---|---|---|---|
| **Compound** | **Protons** | **With Ca²⁺** | **With Mg²⁺** |
| **10** | H3 | +0.10 | +0.20 |
| | H4 | -0.02 | +0.04 |
| | H5 | -0.06 | -0.02 |
| **11** | H4 | +0.08 | +0.10 |
| | CH₃ (3) | +0.02 | +0.04 |
| | CH₃ (5) | +0.06 | +0.08 |
| **12** | H3 | +0.10 | +0.14 |
| | H4 | +0.02 | +0.03 |
| | H5 | +0.02 | +0.04 |
| | H6 | +0.02 | +0.04 |
| | H7 | -0.02 | -0.04 |
| **13** | H3 | -0.10 | -0.04 |
| | H4 | -0.06 | -0.06 |
| | H5 | -0.02 | +0.02 |
| | H6 | -0.02 | +0.02 |
| | H7 | +0.07 | +0.20 |

It was observed in all the complexones that, on adding Ca²⁺ or alternatively Mg²⁺, the chemical shift of the different azole protons is similar at both pH values studied. However, larger modifications in the chemical shift (Δδ) of the protons which were studied are produced at the more basic pH value. In sodium N-[2-(1-pyrazolyl)ethyl]iminodiacetate **10,** the value of the chemical shift of the protons H3, H4 and H5 varied between 0.02 and 0.08 ppm following the addition of metal ions to the medium, both for Ca²⁺ and for Mg²⁺, H3 being somewhat more sensitive to the chelation of magnesium than to that of calcium, and H4 and H5 to that of calcium.

As regards sodium N-[2-(3,5-dimethyl-1-pyrazolyl)ethyl]iminodiacetate, **11,** the azole proton H4 underwent a rather more appreciable modification of its δ value at basic pH than at neutral pH. A smaller increase in the value of the chemical shift occurs in the methyl groups CH₃(3) and CH₃(5). Both these protons and the previous one are slightly more sensitive to Mg²⁺ than to Ca²⁺.

The azole proton H3 of compound **12,** sodium N-[2-(1-indazolyl)ethyl]iminodiacetate, detects the chelation with the cations at pH ∼7, although the change in its chemical shift is very small: 0.04 ppm both for Ca²⁺ and for Mg²⁺. However, at the more basic pH, the sensitivity of this proton is considerably greater. Something similar occurs with the remaining azole protons of this compound. It should be pointed out in this complexone that the proton H7 of the latter is the only one of all those studied in this work which shifts downfield throughout the pH titration, though this deshielding is very small. This same behaviour is observed in the protons H5, H6 and H7 of this molecule on adding calcium, where they also shift downfield as the proton concentration of the medium decreases.

Lastly, bearing in mind that in compound **13,** sodium N-[2-(2-indazolyl)ethyl]iminodiacetate, the azole proton H3 does not undergo a modification in its chemical shift over the entire pH range, the variations produced in the resonance of this compound after adding the cations to the medium are due only and exclusively to the chelation of these cations with the complexone. This variation is fixed irrespective of the pH value: 0.1 ppm for the chelation with calcium and 0.04 ppm for magnesium. The value of Δδ in the protons H4, H5, H6 and H7 in the presence of Ca²⁺ and Mg²⁺ fluctuates between 0.02 ppm and 0.10 ppm, except for H7 at basic pH which deshields by 0.2 ppm. In this complexone, it should be emphasized that, at physiological pH, one of the protons is fairly sensitive to the presence of calcium and less to that of magnesium, H3, and in another proton precisely the opposite occurs, H7. This represents a great advantage, since it is desirable for there to be protons of different sensitivity with respect to bivalent cations in the same molecule.

Finally, the results obtained in this study suggest that the presence of only one iminodiacetate group does not produce, following chelation of the latter with metal ions, great effects on the resonances of the azole protons. Nevertheless, these effects, though small, may be useful from a biological standpoint, in particular in compound 13. In this case, the possibility of having at one's disposal, in the same molecule, selective detectors for Ca²⁺ and Mg²⁺ merits special attention.

### Synthesis of 1(2)-(2-bromoethyl)azoles

The azoles used as starting materials, as well as the dibromoethane employed, were commercial and were used without further purification.

### GENERAL METHOD A

40% sodium hydroxide, the azole, the catalyst, tetrabutylammonium bromide (TBAB) and dibromoethane in a mole ratio (3:1:1/40:8 or 10) are introduced into a round-bottomed flask equipped with a reflux condenser. The mixture is kept stirring at room temperature for 24 hours. The organic phase is decanted, concentrated and purified by column chromatography (silica gel and the appropriate solvent(s)) or by vacuum distillation.

### GENERAL METHOD B

40% sodium hydroxide, the azole, the catalyst, tetrabutylammonium bromide (TBAB) and 1,2-dibromoethane in a mole ratio (3:1:1/40:10) are introduced into a round-bottomed flask equipped with a reflux condenser. The mixture is heated on an oil bath at a temperature of 120-130°C for one hour. The organic phase is decanted, concentrated and purified by column chromatography (silica gel and the appropriate solvent(s)).

### 1-(2-Bromoethyl)pyrazole (1)²⁴

GENERAL METHOD A (from 29.4 to 117.6 mmol of azole). Boiling point: 38-40°C (0.01 mm Hg). Yld. (%) = 71. **IR** (film): 3110, 3020, 2970, 2920, 1510, 1445, 1410, 1395, 1290, 1260, 1215, 1125, 1090, 1050, 965, 915, 880 755 cm⁻¹. **Mass:** m/z 176 (M+1, 11%), 174 (M-1, 11%), 95 (31%), 81 (41%), 69 (8%), 68 (100%), 54 (15%), 53 (17%), 52 (8%). **¹H NMR** (CDCl₃): 7.56 (d, 1 H, ³J₃₄ = 1.6 Hz, H3), 7.46 (d, 1 H, ³J₄₅ = 2.1 Hz, H5), 6.26 (apparent triplet, 1 H, H4), 4.51 (t, 2 H, ³J = 6.4 Hz, CH₂-N), 3.72 (t, 2 H, ³J = 6.4 Hz, CH₂-Br). **¹³C NMR** (CDCl₃): 139.8 (d, ¹J_{C3H3} = 185.2 Hz, C3), 129.7 (d, ¹J_{C5H5} = 186.4 Hz, C5), 105.1 (d, ¹J_{C4H4} = 177 Hz, C4), 52.9 (t, ¹J = 141.6 Hz, CH₂-N), 30.2 (t, ¹J = 153.6 Hz, CH₂-Br).

### 1-(2-Bromoethyl)-3,5-dimethyipyrazole (2)²⁶

GENERAL METHOD B (for 14.0 mmol of azole). Boiling point: 89-90°C (4 mmHg). Chromatographic eluent: ethyl acetate. Yld. (%): 42. **IR** (film): 3130, 3030, 2960, 2920, 2870, 1550, 1455, 1420, 1385, 1300, 1260, 1215, 1145, 1030, 975, 880, 780 cm⁻¹. **Mass:** m/z: 204 (M+1, 11%), 203 (M, 1%), 202 (M-1, 11%), 123 (4%), 109 (43%), 97 (6%), 96 (100%), 95 (35%), 82 (6%), 81 (6%), 68 (11%), 55 (6%). **¹H NMR** (CDCl₃): 5.79 (s, 1 H, H4), 4.31 (t, 2 H, ³j = 6.7 Hz, CH₂-N), 3.68 (t, 2 H, ³J=6.7 Hz, CH₂-Br), 2.27 (s, 3 H, CH₃-5-), 2.21 (s, 2 H, CH₃-3-). **¹³C NMR** (CDCl₃): 148.0 (s, C3), 139.2 (s, C5), 104.9 (d, ¹J_{C4H4} = 172.6 Hz, C4), 49.3 (t, ¹J = 140.7 Hz, CH₂-N), 30.2 (t, ¹J = 153.4 Hz, CH₂-Br), 13.3 (c, ¹J = 126.9 Hz, CH₃-3-), 10.8 (q, ¹J = 128.5 Hz, CH₃-5-).

### 1-(2-Bromoethyl)indazole (3)

GENERAL METHOD A (from 4.24 to 17.0 mmol of azole). Chromatographic eluent: hexane/ethyl acetate, 6:4. Yld. (%): 44. **IR** (film): 3080, 3060, 2980, 2940, 1645, 1615, 1500, 1465, 1435, 1420, 1315, 1300, 1275, 1230, 1210, 1160, 1010, 910, 850, 835, 755, 745 cm⁻¹. **Mass:** m/z: 226 (M+1, 17%), 225 (M, 1%), 224 (M-1, 17%), 132 (9%), 131 (100%), 118 (16%), 104 (11%), 103 (15%), 89 (8%), 77 (21%), 63 (17%), 51 (9%). **¹H NMR** (CDCl₃): 8.04 (d, 1 H, ⁵J₃₇ = 0.8 Hz, H3), 7.78 (ddd, 1 H, ³J₄₅ = 8.0 Hz, ⁴J₄₆ = 0.9 Hz, ⁵J₄₇ = 0.9 Hz, H4), ∼7.45 (m, 2 H, H6 and H7), 7.20 (ddd, 1 H, ³J₄₅ = 8.0 Hz, ³J₅₆ = 6.2 Hz, ⁴J₅₇ = 1.8 Hz, H5), 4.76 (t, 2 H, ³J = 6.8 Hz, CH₂-N), 3.79 (t, 2 H, ³J = 6.8 Hz, CH₂-Br). **¹³C NMR** (CDCl₃): 139.5 (s, C7ₐ), 133.7 (d, ¹J_{C3H3} = 189.9 Hz, C3), 126.3 (d, ¹J_{C6H6} = 165.7 Hz, C6), 123.6 (s, C3ₐ), 120.9 (d, ¹J_{C5H5} = 154.9 Hz, C5), 120.6 (d, ¹J_{C4H4} = 165.4 Hz, C4), 49.7 (t, ¹J = 141.4 Hz, CH₂-N), 29.4 (t, ¹J = 153.5 Hz, CH₂-Br). GENERAL METHOD B (from 4.24 to 17.0 mmol of azole). Chromatographic eluent: hexane/ethyl acetate, 6:4. Yld. (%): 46.

### 2-(2-Bromoethyl)indazole (4)

GENERAL METHOD A (from 4.24 to 17.0 mmol of azole). Chromatographic eluent: hexane/ethyl acetate, 6:4. Yld. (%): 20. **IR** (film): 3120, 3060, 2970, 2920, 1625, 1510, 1470, 1425, 1380, 1350, 1310, 1265, 1160, 1140, 785, 760 cm⁻¹. **Mass:** m/z: 226 (M+1, 11%), 225 (M, 1%), 224 (M-1, 11%), 119 (9%), 118 (100%), 103 (4%), 91 (9%), 89 (9%), 77 (10%), 63 (16%), 51 (5%). **¹H NMR** (CDCl₃): 8.00 (d, 1 H, ⁵J₃₇ = 0.8 Hz, H3), 7.70 (dddd, 1 H, ³J₆₇ = 7.3 Hz, ⁴J₅₇ = 0.9 Hz, ⁵J₄₇ = 1.0 Hz, ⁵J₃₇ = 0.8 Hz, H7), 7.66 (ddd, 1 H, ³J₄₅ = 7.3 Hz, ⁴J₄₆ = 1.0 Hz, ⁵J₄₇ = 1.0 Hz; H4), 7.30 (ddd, 1 H, ³J₆₇ = 7.3 Hz, ³J₅₆ = 6.7 Hz, ⁴J₄₆ = 1.0 Hz, H6), 7.10 (ddd, 1 H, ³J₄₆ = 7.3 Hz, ³J₅₆ = 6.7 Hz, ⁴J₅₇ = 0.9 Hz, H5), 4.76 (t, 2 H, ³J = 6.3 Hz, CH₂-N), 3.79 (t, 2 H, ³J = 6.3 Hz, CH₂-Br). **¹³C NMR** (CDCl₃): 149.0 (s, C7ₐ), 126.1 (d, ¹J_{C6H6} = 160.6 Hz, C6), 123.6 (d, ¹J_{C3H3} = 189.9 Hz, C3), 121.6 (d, ¹J_{C5H5} = 159.7 Hz, C5), 121.2 (s, C3a), 120.1 (d, ¹J_{C4H4} = 156.4 Hz, C4), 117.0 (d, ¹J_{C7H7} = 167.0 Hz, C7), 54.5 (t, ¹J = 142.6 Hz, CH₂-N), 29.4 (t, ¹J = 155.6 Hz, CH₂-Br). GENERAL METHOD B (from 4.24 to 17.0 mmol of azole). Chromatographic eluent: hexane/ethyl acetate, 6:4. Yld. (%): 14.

### Synthesis of methyl N-[2-(1(2)-azolyl)ethyl]iminodiacetates or methyl N-(methoxycarbonylmethyl)-N-[2-(1(2)-azolyl)ethyl]glycinates

The bromoethylazoles synthesized previously and purified were employed. The methyl iminodiacetate used was obtained from commercial iminodiacetonitrile according to the method described in the literature²⁷.

### GENERAL METHOD

The bromoethylazole and methyl iminodiacetate in a mole ratio (1:2) are introduced into a round-bottomed flask equipped with a reflux condenser and calcium chloride tube. The mixture is heated on an oil bath at a temperature of 110°C for a period of time from 2.30 to 4.30 hours. The reaction mixture is cooled and extracted with methylene chloride, concentrated and purified by distillation and/or column chromatography (silica gel and the appropriate solvent(s)).

### Methyl N-[2-(1-pyrazolyl)ethyl]iminodiacetate or methyl N-(methoxycarbonylmethyl)-N-[2-(1-pyrazolyl)ethyl]glycinate (5)

GENERAL METHOD (from 2.24 to 6.7 mmol of 1-(2-bromoethyl)pyrazole). Reaction time: 2.5 h. Chromatographic eluent: hexane/ethanol, 8:2. Boiling point: 128-130°C (0.05-0.01 mm Hg). Yld. (%): 55. **IR** (film): 3140, 3120, 3000, 2960, 2840, 1740, 1510, 1435, 1395, 1365, 1280, 1205, 1180, 1145, 1090, 1060, 1040, 1010, 880, 760 cm⁻¹. **Mass:** m/z: 256 (M+1, 4%), 255 (M, 13%), 196 (28%), 188 (6%), 187 (50%), 174 (55%), 146 (40%), 128 (70%), 116 (27%), 100 (17%), 95 (17%), 94 (14%), 81 (18%), 69 (14%), 68 (37%), 59 (18%), 56 (25%), 54 (18%), 45 (100%), **¹H NMR** (CDCl₃): 7.53 (d, 1 H, ³J₃₄ = 1.8 Hz, H3), 7.50 (d, 1 H, ³J₄₅ = 2.0 Hz, H5), 6.22 (apparent triplet, 1 H, H4), 4.24 (t, 2 H, ³J = 6.2 Hz, CH₂-azole), 3.19 (t, 2 H, ³j = 6.2 Hz, CH₂-N-). **¹³C NMR** (CDCl₃): 171.6 (s, 2 C, CO), 139.3 (d, ¹J_{C3H3} = 186.9 Hz, C3), 130.0 (d, ¹J_{C5H5} = 190.9 Hz, C5), 105.1 (d, ¹J_{C4H4} = 176.3 Hz, C4), 55.3 (t, 2 C, ¹J = 136.5 Hz, CH₂-CO-), 54.7 (t, ¹J = 135.8 Hz, CH₂-N-), 51.5 (q, 2 C, ¹J = 147.2 Hz, CH₃), 51.2 (t, ¹J = 139.6 Hz, CH₂-azole). Picrate: melting point: 91-93°C. Elemental analysis for **C₁₇H₂₀H₆O₁₁:** Calculated: %C = 42.34, %H = 4.07, %N = 17.08. Found: %C = 42.31, %H =4.10,%N=17.11.

### Methyl N-[2-(3,5-dimethyl-1-pyrazolyl)ethyl]iminodiacetate or methyl N-(methoxycarbonylmethyl)-N-[2-(3,5-dimethyl-1-pyrazolyl)ethyl]glycinate (6)

GENERAL METHOD (from 2.46 to 5.55 mmol of 1-(2-bromoethyl)-3,5-dimethylpyrazole). Reaction time: 3.5 h. Boiling point: 140-142°C (0.05-0.01 mm Hg). Yld. (%): 65. **IR:** 3130, 3000, 2960, 2930, 2870, 2220, 1740, 1550, 1435, 1385, 1260, 1200, 1180, 1170, 1120, 1050, 1020, 915, 775, 730 cm⁻¹. **Mass:** m/z: 283 (M, 5%), 224 (13%), 187 (28%), 174 (27%), 146 (35%), 128 (55%), 124 (7%), 116 (19%), 114 (6%), 109 (7%), 100 (9%), 97 (19%), 96 (16%), 82 (14%), 68 (6%), 59 (9%), 56 (12%), 54 (7%), 45 (100%). **¹H NMR** (CDCl₃): 5.74 (s, 1 H, H4), 4.07 (t, 2 H, ³J = 6.8 Hz, CH₂-azole), 3.67 (s, 6 H, CH₃), 3.47 (s, 4 H, CH₂-CO-), 3.09 (t, 2 H, ³J = 6.8 Hz, CH₂-N-), 2.24 (s, 3 H, CH₃-5-), 2.18 (s, 3 H, CH₃-3). **¹³C NMR** (CDCl₃): 171.2 (s, 2 C, CO), 147.0 (s, C3), 139.1 (s, C5), 104.4 (d, ¹J_{C4H4} = 172.1 Hz, C4), 55.1 (t, 2 C, ¹J = 136.4 Hz, CH₂-CO-), 54.2 (t, ¹J = 136.6 Hz, CH₂-N-), 51.1 (q, 2C, ¹J = 142.1 Hz, CH₃), 47.5 (t, ¹J = 138.8 Hz, CH₂-azole), 13.0 (q, ¹J = 123.4 Hz, CH₃-3-), 10.5 (q, ¹J = 127.9 Hz, CH₃-5-). Picrate: Melting point: 83-85°C. Elemental analysis for **C₁₉H₂₄N₆O₁₁:** Calculated: %C = 44.53, %H = 4.69, %N = 16.41. Found: %C = 44.58, %H = 4.64, %N = 16.54.

### Methyl N-[2-(1-indazolyl)ethyl]iminodiacetate or methyl N-(methoxycarbonylmethyl)-N-[2-(1-indazolyl)ethyl] glycinate (7)

GENERAL METHOD (for 6 mmol of 1-(2-bromoethyl)indazole). Reaction time: 4.5 h. Boiling point: 160-162°C (0.01 mm Hg). Yld. (%): 56. **IR** (film): 3060, 3000, 2950, 2850, 1740, 1610, 1505, 1470, 1435, 1315, 1200, 1180, 1160, 1120, 1085, 1010, 910, 830, 755, 740 cm⁻¹. **Mass**: m/z: 305 (M, 6%), 246 (11%), 187 (23%), 175 (6%), 174 (72%), 146 (39%), 131 (6%), 128 (17%), 118 (11%), 116 (20%), 115 (6%), 94 (10%), 91 (5%), 77 (14%), 56 (8%), 51 (5%), 45 (100%). **¹H NMR** (CDCl₃): 7.99 (d, 1 H, ⁵J₃₇ = 0.8 Hz, H3), 7.71 (ddd, 1 H, ³J₄₅ = 8.1 Hz, ⁴J₄₆ = 1.0 Hz, ⁵J₄₇ = 1.0 Hz, H4), 7.50 (dddd, 1 H, ³J₆₇ = 8.1 Hz, ⁴J₅₇ = 0.8 Hz, ⁵J₄₇ = 1.0 Hz, ⁵J₃₇ = 0.8 Hz, H7), 7.37 (ddd, 1 H, ³J₆₇ = 8.1 Hz, ³J₅₆ = 6.8 Hz, ⁴J₄₆ = 1.0 Hz, H6), 7.13 (ddd, 1 H, ³J₄₅ = 8.1 Hz, ³J₅₆ = 6.8 Hz, ⁴J₅₇ = 0.8 Hz, H5) 4.54 (t, 2 H, ³J = 6.8 Hz, CH₂-azole), 3.65 (s, 6 H, CH₃), 3.49 (s, 4 H, CO-CH₂-N), 3.26 (t, 2 H, ³J = 6.8 Hz, CH₂-N-). **¹³C NMR** (CDCl₃): 171.1 (s, 2 C, CO), 139.4 (s, C7ₐ), 132.7 (d, ¹J_{C3H3} = 189.3 Hz, C3), 125.8 (d, ¹J_{C6H6} = 160.7 Hz, C6), 123.6 (s, C3ₐ), 120.5 (d, ¹J_{C5H5} = 161.3 Hz, C5), 120.1 (d, ¹J_{C4H4} = 160.9 Hz, C4), 108.9 (d, ¹J_{C7H7} = 163.6 Hz, C7), 55.1 (t, 2 C, ¹J = 137.8, Hz, CH₂-CO-), 53.5 (t, ¹J = 135.8 Hz, CH₂-N-), 51.1 (q, 2 C, ¹J = 147.0 Hz, CH₃), 47.7 (t ¹J = 139.2 Hz, CH₂-azole). Picrate: melting point: 105.5-107.5°C. Elemental analysis for **C₂₁H₂₂N₆O₁₁:** Calculated: %C = 47.19, %H = 4.20, %N = 15.73. Found: %C = 46.80, %H = 4.07, %N = 15.31.

### Methyl N-[2-(2-indazolyl)ethyl]iminodiacetate or methyl N-(methoxycarbonylmethyl)-N-[2-(2-indazolyl)ethyl]glycinate (8)

GENERAL METHOD (for 3.32 mmol of 2-(2-bromoethyl)indazole). Furnace temperature: 250°C (0.01 mm Hg). In a balling furnace. Yld. (%): 60. **IR** (film): 3120, 3060, 3000, 2960, 1735, 1620, 1505, 1430, 1375, 1355, 1200, 1180, 1160, 1055, 1010, 980, 905, 785, 760, 745 cm⁻¹. **Mass:** m/z: 305 (M, 10%), 246 (11%), 187 (31%), 174 (26%), 146 (28%), 128 (57%), 119 (8%), 118 (36%), 116 (13%), 114 (10%), 100 (9%), 94 (6%), 91 (6%), 89 (6%), 77 (9%), 59 (10%), 56 (7%), 54 (5%), 45 (100%). **¹H NMR** (CDCl₃): 8.11 (d, 1 H, ⁵J₃₇ = 0.9 Hz, H3), 7.69 (dddd, 1 H, ³J₆₇ = 7.7 Hz, ⁴J₅₇ = 1.0 Hz, ⁵J₄₇ = 1.2 Hz, ⁵J₃₇ = 0.9 Hz, H7), 7.65 (ddd, 1 H, ³J₄₅ = 6.3 Hz, ⁴J₄₆ = 1.2 Hz, ⁵J₄₇ = 1.2 Hz, H4), 7.27 (ddd, 1 H, ³J₅₆ = 8.3 Hz; ³J₆₇ = 7.7 Hz, ⁴J₄₆ = 1.2 Hz, H6), 7.06 (ddd, 1 H, ³J₅₆ = 8.3 Hz, ³J₄₅ = 6.3 Hz, ⁴J₅₇ = 1.0 Hz, H5), 4.52 (t, 2 H³J = 6.1 Hz, CH₂-azole), 3.65 (s, 6 H, CH₃), 3.46 (s, 4 H, CH₂-CO-), 3.35 (t, 2 H, ³J = 6.1 Hz, CH₂-N-). **¹³C NMR** (CDCl₃): 171.5 (s, 2 C, CO), 148.7 (s, C7ₐ)**,** 125.7 (d, ¹J_{C6H6} = 165.2 Hz, C6), 124.0 (d, ¹J_{C3H3} = 190.3 Hz, C3), 121.5 (s, C3ₐ), 121.2 (d, ¹J_{C4H4} = 159.4 Hz, C4), 120.1 (d, ¹J_{C5H5} = 165.2 Hz, C5), 117.0 (d, ¹J_{C7H7} = 160 Hz, C7), 55.5 (t, 2 C, ¹J = 137.6 Hz, CH₂-CO-), 55.0 (t, ¹J = 136.5 Hz, CH₂-N-), 52.7 (t, ¹J = 141.2 Hz, CH₂-azole), 51.4 (q, 2 C, ¹J = 147.2 Hz, CH₃). Picrate: melting point: 140.6-142.4°C. Elemental analysis for **C₂₁H₂₂N₆O₁₁:** Calculated: %C = 47.19, %H = 4.20, %N = 15.73. Found: %C = 47.40, %H = 4.62, %N = 15.61.

### Hydrolysis of methyl N-[2-(1 (2)-azolyl)ethyl]iminodiacetates or methyl N-(methoxycarbonylmethyl)-N-[2-(1(2)-azolyl)ethyl]glycinates

The esters previously synthesized and purified were employed. The HCl and NaOH used were commercial and were used without further purification.

### GENERAL METHOD A: ACID HYDROLYSIS

The ester and 2 N HCl in a mole ratio (1:18) are introduced into a round-bottomed flask equipped with a reflux condenser. The mixture is heated on an oil bath at a temperature of 100°C for 4 hours. The corresponding acid hydrochloride is concentrated and purified by recrystallization (employing the appropriate solvent(s)).

### GENERAL METHOD B: BASIC HYDROLYSIS

The ester and 0.6% NaOH in a mole ratio (1:2) are introduced into a round-bottomed flask equipped with a reflux condenser. The mixture is kept stirring at room temperature for at least 24 hours. It is concentrated and the disodium salt is obtained.

### N-[2-(1-Pyrazolyl)ethyl]iminodiacetic acid hydrochloride or N-(carboxymethyl)-N-[2-(1-pyrazolyl)ethyl]glycine hydrochloride (9)

GENERAL METHOD A (for 0.7 mmol of ester). Melting point: 142.5-144.5°C. Recrystallization solvent: EtOH/Et₂O- Yld. (%): ∼100. **¹H NMR** (D₂O): 7.70 (d, 1 H, ³J₄₅ = 2.4 Hz, H5), 7.61 (d, 1 H, ³J₃₄ = 2.2 Hz, H3), 6.31 (apparent triplet, 1 H, H4), 4.53 (t, 2 H, ³J = 6.0 Hz, CH₂-azole), 3.70 (t, 2 H, ³J = 6.0 Hz, CH₂-N-). Elemental analysis for **C₉H₁₃N₃O₄.HCl:** Calculated: %C = 40.98, %H = 5.35, %N = 15.93. Found: %C = 41.07, %H = 5.28, %N = 15.50.

### Sodium N-[2-(1-pyrazolyl)ethyl]iminodiacetate or N-(carboxymethyl)-N-[2-(1-pyrazolyl)ethyl]glycine disodium salt (10)

GENERAL METHOD B (from 4 to 40 mmol of ester). Yld. (%): ∼100. **IR** (KBr): 3130, 3100, 2960, 2940, 2870, 2840, 1730, 1600, 1505, 1425, 1395, 1345, 1300, 1205, 1180, 1140, 1115, 1090, 1055, 1030, 905, 870, 775, 740 cm⁻¹. **¹H NMR** (D₂O): 7.63 (dd, 1 H, ³J₄₅ = 1.8 Hz, ⁴J₃₅ = 0.6 Hz, H5), 7.49 (dd, 1 H, ³J₃₄ = 1.5 Hz, ⁴J₃₅ = 0.6 Hz, H3), 6.26 (apparent triplet, 1 H, H4), 4.21 (t, 2 H, ³J = 6.7 Hz, CH₂-azole), 3.09 (s, 4 H, CH₂-CO-), 2.98 (t, 2 H, ³J = 6.7 Hz, CH₂-N-).

### Sodium N-[2-(3,5-dimethyl-1-pyrazolyl)ethyl]iminodiacetate or N-(carboxymethyl)-N-[2-(3,5-dimethyl-1-pyrazolyl)ethyl]glycine disodium salt (11)

GENERAL METHOD B (from 0.353 to 0.706 mmol of ester). Yld. ∼100. **IR** (KBr): 2980, 2950, 2920, 2880, 2830, 1600, 1550, 1420, 1350, 1300, 1260, 1230, 1200, 1140, 1095, 1040, 1010, 1000, 920, 940, 900, 825, 770 cm⁻¹. **¹H NMR** (D₂O): 5.85 (s, 1 H, H4), 4.04 (t, 2 H, ³J = 7.5 Hz, CH₂-azole), 3.16 (s, 4 H, CH₂-CO-), 2.90 (t, 2 H, ³J = 7.5 Hz, CH₂-N-), 2.17 (s, 3 H, CH₃-5-), 2.07 (s, 3 H CH₃-3-).

### Sodium N-[2-(1-indazolyl)ethyl]iminodiacetate or N-(carboxymethyl)-N-[2-(1-indazolyl)ethyl]glycine disodium salt (12)

GENERAL METHOD B (for 0.492 mmol of ester). Yld. ∼100. **IR** (KBr): 3090, 3050, 2940, 2920, 2870, 2830, 1600, 1460, 1430, 1350, 1330, 1315, 1300, 1250, 1210, 1180, 1140, 1070, 1005, 940, 905, 880, 830, 795, 740 cm⁻¹. **¹H NMR** (D₂O): 8.02 (d, 1 H, ⁵J₃₇ = 1.0 Hz, H3), 7.75 (ddd, 1 H, ³J₄₅ = 8.1 Hz, ⁴J₄₆ = 1.1 Hz, ⁵J₄₇ = 1.0 Hz, H4), 7.57 (dddd, 1 H, ³J₆₇ = 7.8 Hz, ⁴J₅₇ = 0.9 Hz, ⁵J₄₇ = 1.0 Hz, ⁵J₃₇ = 1.0 Hz, H7), 7.43 (ddd, 1 H, ³J₆₇ = 7.8 Hz; ³J₅₆ = 6.8 Hz, ⁴J₄₆ = 1.1 Hz, H6), 7.14 (ddd, 1 H, ³J₄₅ = 8.1 Hz; ³J₅₆ = 6.8 Hz, ⁴J₅₇ = 0.9 Hz, H5) 4.46 (t, 2 H, ³J = 7.0 Hz, CH₂-azole), 3.14 (s, 4 H, CH₂-CO-), 3.04 (t, 2 H, ³J = 7.0 Hz, CH₂-N-).

### Sodium N-[2-(2-indazolyl)ethyl]iminodiacetate or N-(carboxymethyl)-N-[2-(2-indazolyl)ethyl]glycine disodium salt (13)

GENERAL METHOD B (for 0.492 mmol of ester). Yld. ∼100. **IR** (KBr): 3110, 3070, 2950, 2920, 2870, 2830, 1600, 1510, 1430, 1350, 1300, 1225, 1160, 1140, 1045, 1005, 980, 940, 905, 840, 775, 755 cm⁻¹. **¹H NMR** (D₂O): 8.29 (d, 1 H, ⁵J₃₇ = 0.9 Hz, H3), 7.71 (ddd, 1 H, ³J₄₅ = 8.4 Hz, ⁴J₄₆ = 1.1 Hz, ⁵J₄₇ = 1.0 Hz, H4), 7.57 (dddd, 1 H, ³J₆₇ = 8.7 Hz, ⁴J₅₇ = 1.0 Hz, ⁵J₄₇ = 1.0 Hz, ⁵J₃₇ = 0.9 Hz, H7), 7.31 (ddd, 1 H, ³J₆₇ = 8.7 Hz, ³J₅₆ = 6.6 Hz, ⁴J₄₆ = 1.1 Hz, H6), 7.08 (ddd, 1 H, ³J₄₅ = 8.4 Hz, ³J₅₆ = 6.6 Hz, ⁴J₅₇ = 1.0 Hz, H5), 4.74 (t, 2 H, ³J = 6.8 Hz, CH₂-azole), 4.51 (t, ³J = 6.8 Hz, CH₂-N).

### pH titration of the probes. (Hydrochlorides or disodium salts)

The probes synthesized previously as acid hydrochlorides or as disodium salts were employed. NaOH solutions were prepared using 99.98% D₂O from the commercial reagent without further purification. DCI solutions were prepared from commercial concentrated DCI and likewise employing D₂O.

### GENERAL METHOD

Twelve 25 mM solutions of each of the probes, **10-13,** are prepared in 99% D₂O (the same number of solutions as pH points which it is desired to analyse). 1 M DCI is used to attain acid pH values and 1 M NaOD for the basic pH values. The final volume of the samples should be 1 mL. In each sample the requisite pH value is fixed, ensuring that the range from 1 to 12 is covered and that there is a difference of one unit between one sample and the next. The ¹H NMR spectrum of the samples is recorded at the different pH values. For all the protons of each of the probes, their chemical shift is plotted against the pH in order to obtain the titration curve.

### Measurements of chelation with CaCl₂ and MgCl₂. (Disodium salts)

The probes synthesized previously as disodium salts were employed. NaOH solutions as well as those of all the ions employed were prepared using 99.98% D₂O in all cases, from commercial reagents without further purification. DCI solutions were prepared from commercial DCI and likewise employing D₂O.

Ca²⁺ and Mg²⁺ solutions were prepared from 98% CaCl₂.2H₂O and 99.995% MgCl₂.6H₂O, respectively, and D₂O as solvent.

### GENERAL METHOD

Two 25 mM solutions of each of the probes, **10-13,** are prepared in 99.98% D₂O at two different pH values: one lying between 11 and 12 (as close as possible to 11) and the other between 9 and 10 (as close as possible to 9). The pH is adjusted in a similar manner to that in the previous section. 50 mL of 1 M CaCl₂ or MgCl₂ solutions are added. The final pH of the samples should in one case be close to 9 and in the other close to physiological pH (∼7), and is adjusted if necessary. The final volume should be 1 mL. The ¹H NM R spectrum of each sample is recorded. The chemical shift of all the protons of the probes is noted at both pH values analysed, and for all the ions added.

### Bibliographic References

1. Andrew, E. R.; Byder, G.; Griffith, J.; Iles, R.; Styles, P.; Clinical Magnetic Resonance Imaging and Spectroscopy. John Wiley and Sons, N. Y, 1990.
2. Cerdán, S.; Seelig, J.; Ann. Rev. Biophys. Chem., 1990, 19, 43**.**
3. a) Szwergold, B.; Ann. Rev. Physiol., 1992, 54, 775; b) Gil, M. S.; Zaderenko, P.; Cruz, F.; Cerdán, S.; Balles-teros, P.; Bioorg. Med. Chem., 1994, 2, 305.
4. a) Carafoli, E.; Methods in Enzymol., 1988, 57, 3; b) Denton, R. M.; McCormack, J. G.; Ann. Rev. Physiol., 1990, 52,451.
5. Vergara, J.; Tzien, R. Y; Delay, M.; Proc. Natl. Acad. Sci. U.S.A., 1985, 82, 6352.
6. Berridge, M. J.; Ann. Rev. Biochem., 1987, 56, 159*.*
7. Watson, S. P.; Ruggiero, M.; Abrahamns, S. L.; Lapetina, E. G.; J. Biol. Chem., 1986, 261, 5368.
8. Brass, L. F.; Joseph, S. K.; J. Biol. Chem., 1985, 260, 15172.
9. Lapetina, E. G.; Watson, S. P.; Cuatrecasa, P.; Proc. Natl. Acad. Sci. U.S.A., 1984, 81, 7531.
10. Carafoli, E.; Caroni, P.; Chiesi, M.; Famulski, K.; Metabolic Compartmentation. H. Sies, Ed., Academic Press, 1982, p. 521.
11. Wilson, D. B., Connolley, T M.; Bross, T. E.; Majerus, P. N.; Sherman, W. R.; J. Biol. Chem., 1985, 260, 13946*.*
12. a) Carafoli, E.; Ann. Rev. Biochem., 1987, 56, 395; b) Meldolesi, J.; Pozzan, T; Exp. Cell. Res., 1987, 171, 271.
13. Tsien, R. Y; Methods in Cell Biology, 1989, 30, 127.
14. McCormack, J. G.; Cobbold, P. H.; Cellular Calcium: A Practical Approach. IRL Press. Oxford University Press. Oxford. 1991.
15. Anderegg, G.; Complexones in Comprehensive Coordination Chemistry, Wilkinson, G.; Gillard, R. D.; McClev-erty, J. A. Editors. Vol 2. Pergamon Press, N. Y, 1987, p. 777.
16. Yoon, P. S.; Sharp, R. R.; Biochemistry, 1985, 24, 7269.
17. Tsien, R. Y; Biochemistry, 1980, 19, 2396.
18. Smith, G. A.; Hesketh, R. T; Metcalfe, J. C.; Feeney, J.; Morris, P. G.; Proc. Natl. Acad. Sci. U.S.A., 1983, 80, 7178.
19. Rabenstein, D. L.; Millis. K. K.; Strauss, E. J.; Anal. Chem., 1988, 60, 1380A*.*
22. Dehmlow, E. V ; Dehmlow, S. S. Phase Transfer Catalysis. VCH. 1993.
23. Elguero, J.; Claramunt, R. M.; Garcerán, R.; Heterocycles, 1985, 24, 2233.
24. Canty, A. J.; Honeymnan, R. T J. Organomet. Chem., 1990. 387, 247.
25. Torres, J.; Lavandera, J. L.; Cabildo, P.; Claramunt, R. M.; Elguero, J.; J. Heterocycl. Chem., 1988, 25, 771.
26. Ochi, H.; Miyasaka, T.; Arakawa, K. Yakugaku Zasshi, 1978, 98 (2), 165. Chem. Abst., 1978, 89: 24213k
27. Koelsch, C. F; Robinson, F M. J. Org. Chem., 1956, 21, 1211.
28. Schwarzenbach, G.; Anderegg, G.; Schneider, W.; Senn, H.; Helv. Chim. Acta, 1955, 38, 1147.

## Claims

1. Complexones of the N-[2-(1(2)-azolyl)ethyl]iminodiacetic acid type, synthesis, analytical study and biological applications, consisting of methyl N-[2-(1-pyrazolyl)ethyl]iminodiacetate **(5);** methyl N-[2-(3,5-dimethyl-1-pyrazolyl)ethyl]iminodiacetate **(6);** methyl N-[2-(1-indazolyl)ethyl]iminodiacetate **(7)**; methyl N-[2-(2-indazolyl)ethyl]iminodiacetate **(8);** N-[2-(1-pyrazolyl)ethyl]iminodiacetic acid hydrochloride **(9);** sodium N-[2-(1-pyrazolyl)ethyl]iminodiacetate **(10);** sodium N-[2-(3,5-dimethyl-1-pyrazolyl)ethyl]iminodiacetate **(11);** sodium N-[(2-(1-indazolyl)ethyl]iminodiacetate **(12);** sodium N-[2-(2-indazolyl)ethyl]iminodiacetate **(13), characterized by** the presence of an iminodiacetic acid or ester unit linked to the azole nitrogen via a chain of two carbon atoms.

2. Complexones of the N-[2-(1(2)-azolyl)ethyl]iminodiacetic acid type, synthesis, analytical study and biological applications, according to Claim 1, **characterized by** their complexing capacity with respect to Ca²⁺ and Mg²⁺ ions and other bivalent cations.

3. Complexones of the N-[2-(1(2)-azolyl)ethyl]iminodiacetic acid type, synthesis, analytical study and biological applications, according to Claim 1, **characterized by** their complexing capacity with respect to lanthanides and transition metals.

4. Complexones of the N-[2-(1(2)-azolyl)ethyl]iminodiacetic acid type, synthesis, analytical study and biological applications, the production of which may be carried out by means of the alkylation reaction of methyl iminodiacetate with N-bromoethylazoles and subsequent hydrolysis in an acidic or basic medium.

5. Complexones of the N-[2-(1(2)-azolyl)ethyl]iminodiacetic acid type, synthesis, analytical study and biological applications, according to Claim 4, **characterized in that** the procedure may be modified by alternative synthetic routes which involve the use of N-aminoethylazoles as starting materials.

6. Complexones of the N-[2-(1(2)-azolyl)ethyl]iminodiacetic acid type, synthesis, analytical study and biological applications, according to Claim 4, **characterized in that** the procedure may be modified by alternative synthetic routes which involve the use of cyclization processes in the preparation of the N-substituted azole.

7. Complexones of the N-[2-(1(2)-azolyl)ethyl]iminodiacetic acid type, synthesis, analytical study and biological applications, for application in nuclear magnetic resonance (NMR) spectroscopy and imaging employing hydrogen or other nuclei, as extrinsic probes and contrast agents, **characterized in that** they can be used non-invasively and non-destructively in both chemical analysis and clinical diagnosis.

8. Complexones of the N-[2-(1(2)-azolyl)ethyl]iminodiacetic acid type, synthesis, analytical study and biological applications, for application according to Claim 7 as extrinsic probes, **characterized by** their capacity for determining by ¹H NM R spectroscopy the concentration of Ca²⁺, Mg²⁺ or other metal ions in solutions, biological extracts, tissue biopsies, isolated or cultured cells, perfused organs, tissues in vivo, whole animals and human beings.

9. Complexones of the N-[2-(1(2)-azolyl)ethyl]iminodiacetic acid type, synthesis, analytical study and biological applications, for application as extrinsic probes according to Claim 7, **characterized by** their capacity for determining the concentration of Ca²⁺, Mg²⁺ or other metal ions in biological extracts, tissue biopsies, isolated or cultured cells, perfused organs, tissues in vivo, whole animals and human beings, using ¹H NMR imaging technologies including single voxel and multivoxel imaging and chemical shift spectroscopic imaging.

10. Complexones of the N-[2-(1(2)-azolyl)ethyl]iminodiacetic acid type, synthesis, analytical study and biological applications, for application according to Claim 7, **characterized by** their capacity for giving rise to paramagnetic complexes with lanthanides which prove useful as contrast agents in imaging obtained by means of any of the magnetic resonance technologies.
